# EUROPEAN PATENT APPLICATION

(11) **EP 1 731 181 A1**
(43) Date of publication of application: **13.12.2006**
(21) Application number: 05076321.8
(22) Date of filing: 07.06.2005
(51) Int. Cl.: A61M 1/00

(54) **A surgical suction device**

(71) Applicant: Unomedical A/S, 3460 Birkerod (DK)
(72) Inventor: Hansen, Jan Bertholdt, 3060 Espergaerde (DK)
(74) Representative: Elmeros, Claus

(57) **Abstract**

The present invention concerns a surgical suction device (1). It comprises a suction tube (100) having a substantially longitudinally extending internal suction passage (110), terminating at a suction tip section (112) at the distal end thereof and a suction connection section (114) at the proximal end thereof for connection to a suction source. The surgical suction device (1) is integrally blow moulded in a plastic material in such a way, that at least part of it is provided with a bulbous handle section (116) provided with a friction enhancing surface texture extending along at least a part thereof. The provision of a friction enhancing surface texture allows for the utilization of a softer plastic material. Thus, a surgical suction device is provided, which can exhibit a more flexible suction tip section. Further, the friction enhancing surface texture at the same time provides a steadier handhold for the operator during surgery. Since the surgical suction device is integrally blow moulded, the production costs are reduced, also due to the time reduction of the required further processing, such as machining holes. Using a blow moulding process with a softer plastic material allows for keeping the total weight of the device very low, e.g. a standard size Yankauer may be reduced in weight to from 5 to 10 g.

## Description

The present invention relates to a surgical suction device, comprising a suction tube having a substantially longitudinally extending internal suction passage, terminating at a suction tip section at the distal end thereof and a suction connection section at the proximal end thereof for connection to a suction source.

Such surgical suction devices are used for medical, surgical or dental use to remove foreign matter, fluid, blood, pus, mucous, polyps, bone particles, necrotic and cancerous matter and the like (all of which are hereinafter referred to as "bodily fluids"). Such suction devices are normally provided in two types: a suction nozzle comprising a rigid nozzle or a suction catheter comprising an elongated flexible catheter which can be inserted into the body as desired. The nozzle or catheter is normally connected to a suction source via an intermediate member which is connected by connection means to a flexible tube that is connected to the suction source, the connection means being normally in the form of a hollow spigot. In the suction nozzle the intermediate member may comprise a handle that may be integral with the probe and may have a round or quadratic section.

An example of such a surgical suction device is a simple device known as a Yankauer. This device normally consists of nozzle or catheter being tapered at the patient end and formed with a suction opening. In order that the suction can be controlled, a suction control port is provided in the intermediate member leading to the suction passage therethrough and the medical personnel which is operating the suction device can vary the suction force at the tip of the probe by obturating the suction control port to a greater or lesser extent. Optionally, it may further be provided with suction tip side holes in varying sizes and distances from the suction tip in order to provide additional suction capacity for different sizes pools of bodily fluids. For this end, it may further optionally be provided with a sump tip which is a laterally extending basket shaped tip, individually provided with suction tip side holes.

In a similar manner, two types of Yankauers are known having either a rigid nozzle provided with a handle or a flexible catheter or bulbous tube. The rigid Yankauers are nozzles provided with a manipulating handle, which may be transparent or non-transparent and is provided around a tube passage of substantially uniform section diameter, while the flexible Yankauers essentially is a catheter tube being provided with a tube passage, which expands over a bulbous section in the tube providing a hand hold.

Generally, rigid Yankauers are manufactured by injection moulding in hard plastic materials, such as in particular PVC (polyvinyl chloride), where all components of the device are provided integrally, i.e. rigid tube, handle, connector, and optionally a sump tip. Holes such as the suction tip side holes and the suction control port are normally provided in further processing steps.

Rigid Yankauers have a disadvantage, because the tip of it is more able to inflict damage to already exposed and traumatized tissue, as is present in a surgical wound, than is the tip of a flexible Yankauer. Further, a rigid Yankauer provides for a relatively heavy device, which may be difficult to hold for extended periods of time during surgery for the device operators, e.g. such as the surgeon performing the surgery, and thus tiring, which increases the risk of rough nozzle handling which results in a increase risk of tissue damage.

Flexible Yankauers are in general manufactured by horizontal extrusion by processing a tube continuously, where the tube is pushed and drawn horizontally through a cooling basin and a calibration unit before packaging. Optionally, a suction control port, a sump tip and suction tip side holes may be provided in parts by performing assembling and machining processing steps following the extrusion.

Flexible Yankauers have a disadvantage, because due to their flexibility they can be difficult to manipulate for a surgeon or other operator during surgery. Further, the surgery may produce bodily fluids, which are spilled upon the bulbous section, making the bulbous section slippery to handle, said effect being enhanced by the fact that gloves are used.

WO 98/52626 describes a nozzle such as a Yankauer, for a surgical suction apparatus, which nozzle comprises a connector tube in fluid communication with a curved suction tube, having a suction control port provided with a liquid impermeable membrane to filter away e.g. bacteria. It is described, that the connector part and suction part may be integrally moulded, and may be blow moulded in plastics, but the above mentioned technical problems are not solved in relation to this nozzle.

It is further known to manufacture a Yankauer by extruding PVC mixed with EVA (ethylene vinyl acetate) and a plasticizer in order to provide a less rigid tip. However, this is a disadvantage due to the environmental considerations using biologically active materials, as well as is the use of an extrusion process during manufacturing, which necessitates more processing.

Thus, it is the object of the present invention to provide a surgical suction device, such as a Yankauer, which alleviates the above mentioned problems with known devices, and provides a relatively flexible suction section or nozzle in combination with a relatively rigid handle providing a steady grip.

This object is achieved by providing a surgical suction device of the type described in the preamble, wherein the surgical suction device is integrally blow moulded in a plastic material in such a way, that at least part of it is provided with a bulbous handle section provided with a friction enhancing surface texture extending along at least a part thereof.

The provision of a friction enhancing surface texture allows for the utilization of a softer plastic material. Thus, a surgical suction device is provided, which can exhibit a more flexible suction tip section. Further, the friction enhancing surface texture at the same time provides a steadier handhold for the operator during surgery. Hereby, a combination of advantageous features from both prior art rigid and flexible devices is provided, while at the same time alleviating the above mentioned disadvantages of the same. Since the surgical suction device is integrally blow moulded, the production costs are reduced, also due to the time reduction of the required further processing, such as machining holes. Using a blow moulding process with a softer plastic material allows for keeping the total weight of the device very low, e.g. a standard size Yankauer may be reduced in weight to from 5 to 10 g. The device may also be extruded before being blow moulded, and may be machined before or after blow moulding, e.g. for providing holes.

In a preferred embodiment the surgical suction device according to the invention it is integrally injection blow moulded. Thus, fine details like the connector and a sump tip may be moulded during a two step process, reducing the machining time needed.

In a preferred embodiment the surgical suction device according to the invention the friction enhancing surface texture is provided as a plurality of reinforcement ribs in a pattern around the periphery of the bulbous handle section. Different patterns of the reinforcement ribs are conceivable, e.g. transversal, helical, punctiform, and/or elongated shapes, and combinations thereof. Different forms of ribs are conceivable, e.g. thicker material thickness ribs, or ribs provided by applying a different material from the base plastics material, e.g. a plastic material having a higher tensile modulus and/or hardness. Thus, increased handle stability and grip is provided to the device, while retaining the flexibility of the suction tip section and tip.

In a preferred embodiment the surgical suction device according to the invention the friction enhancing surface texture is longitudinally extending grooves provided in the plastic material itself. The longitudinally extending ribs formed as grooves provide an excellent hand grip as well as for an excellent reinforcement of the plastic material used. Further, the shape of the grooves increases bodily fluid spillage removal, leading such spillage downwards and away from the bulbous handle section during use.

In a preferred embodiment of the surgical suction device according to the invention the plastic material is a styrene butadiene copolymer, preferably Styrolux®. Accordingly, a relatively soft plastic material is utilized providing the basis for a selection of surgical suction devices of different flexibilities, sizes, applications and transparencies. Further, the use of styrene butadiene copolymers such as Styrolux® provides a surgical suction device having a reduced biological activity as compared to a surgical suction device made of PVC. Styrene butadiene copolymers further exhibit other advantageous characteristics, such as low density, high durability, reduced hardness, high transparency, adaptability for injection and blow moulding, and good tensile properties. In particular, when using a styrene butadiene copolymer such as Styrolux®, the weight reduction as compared to using PVC may be as large as four fold. Further, clear styrene butadiene copolymers allows for excellent mouldability and printability for durable inscriptions moulded into the device, such as serial numbers, size numbers, name of producer, year of production, type of plastic used, etc.

In a preferred embodiment of the surgical suction device according to the invention the hardness of the plastic material is in the order of from shore 60 to 80, preferably from shore 65 to 70 according to ISO 868 shore D. Selecting this range of hardness provides a relatively flexible surgical suction device. The limits of the interval are chosen due to the fact that shore 80 is corresponding to the lowermost hardness of PVC, and at shore 60 the resulting surgical suction device is deemed too flexible to provide an accurately determined suction area.

In a preferred embodiment of the surgical suction device according to the invention the tensile modulus of the plastic material is in the order of from 1300 to 1700 MPa, preferably from 1400 to 1500 MPa according to ISO 527. Selecting this range of tensile modulus in combination with the selected hardness interval and the friction enhancing surface texture allow for the production of a wide selection of different flexible surgical suction device configurations with excellent flexibility properties of the suction tip section and excellent rigidity at the bulbous handle section. Further, the device is exhibiting advantageous mechanical properties such as high break at strain and shear modulus.

In another embodiment of the surgical suction device according to the invention at least part of the internal suction passage in the bulbous handle section has a diameter, which is larger than that of the remaining part of the suction passage. Thus, a turbulence chamber is formed, reducing the risk of bodily fluids clogging inside the device.

In a preferred embodiment of the surgical suction device according to the invention the material thickness is substantially uniform throughout the extent thereof, in particular in the bulbous handle section provided with friction enhancing surface texture. Thus, the total weight of the surgical suction device may be reduced substantially, which is particularly true, when the material thickness of the bulbous handle section is substantially uniform both in the grooves and outside these along the ribs. This is an approximation, because the material thickness of the bulbous handle section is necessarily slightly thinner than in the suction tip and connection section, which have not been subjected to a blow moulding step during production.

In a preferred embodiment of the surgical suction device according to the invention the plastic material is transparent and has a transmittance of in the order of 80 to 99 %, preferably from 85 to 98 %, and a haze in the order of from 0 to 8%, preferably from 0 to 5% according to ASTM D 1003. Thus, the bodily fluids sucked from during surgery may be inspected during use for a determination of the amount and condition of these for a regulation of effectiveness and performance of the device and selected suction position in the wound. Further, a low haze property is an advantage because of the resulting low reflectance, which reduces device glare from reflected theatre light.

In a preferred embodiment of the surgical suction device according to the invention it is a Yankauer provided with a bend at the suction tip section, preferably in the interval from 1 to 180°, and a suction control port in the handle section. The surgical suction device is advantageously a Yankauer for the provision of a more controllable suction force and position at the suction tip of the device.

In another embodiment of the surgical suction device according to the invention it is further provided with at least one suction tip side hole at a predetermined position in the suction tip section. Accordingly, a deeper pool of bodily fluids may be sucked up faster, providing a larger suction area of the device.

In another embodiment of the surgical suction device according to the invention it is further provided with an externally ribbed connector in the suction connection section. This provides for an improved stability of connection to a suction source.

In another embodiment of the surgical suction device according to the invention it is further provided with a sump tip at the suction tip section provided with at least one suction tip side hole. Thus, combined with the flexibility of the device, in particular in the suction tip section, the risk of damage inflicted by the suction tip is substantially reduced, while the suction area of the device is increased.

The invention will be described in more detail with reference to the accompanying drawings, in which:
- Fig.1A and 1B: are views of a first embodiment of the surgical suction device according to the invention, where Fig. 1A is a top view of said device and Fig. 1B is a section along the line A-A in Fig. 1A;
- Fig.2A and 2B: are views of a second embodiment of the surgical suction device according to the invention, where Fig. 2A is a top view of said device and Fig. 2B is a section along the line B-B in Fig. 2A; and
- Fig. 3: is a perspective view of the embodiment of the surgical suction device of Fig. 1A.

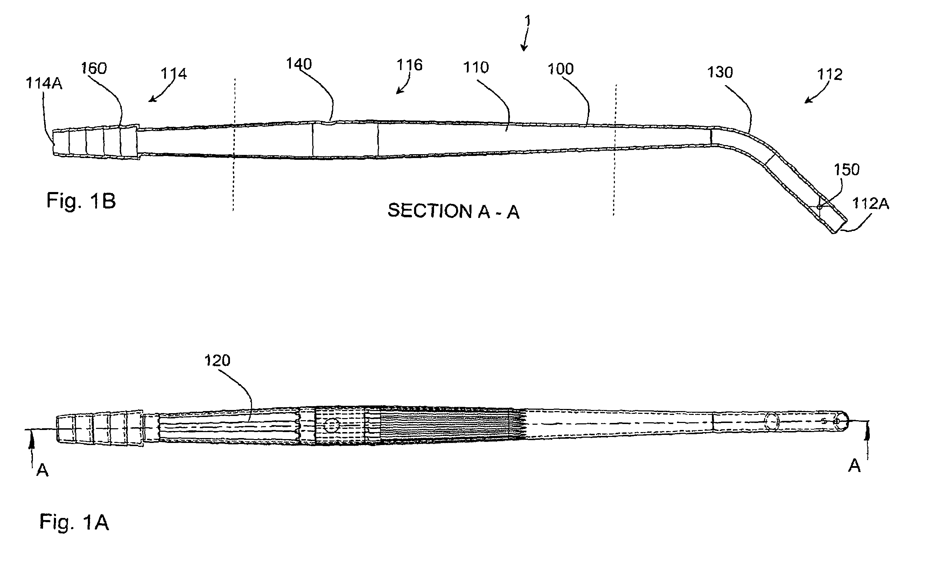

In fig. 1A is shown a first embodiment of the present invention of a surgical suction device 1, of which a section along the line A-A is shown in fig. 1B. The device 1 comprises a suction tube 100 having a substantially longitudinally extending internal suction passage 110, which terminates at a suction tip section 112 and a suction tip 112A at the distal end thereof and a suction connection section 114 and a connection end 114A at the proximal end thereof for connection to a suction source (not shown).

The surgical suction device 1 further comprises a bulbous handle section 116, which as is shown in fig. 1A is provided with a friction enhancing surface texture. The friction enhancing surface texture are longitudinally extending grooves 120 provided in the plastic material itself. They form a plurality i.e. ten reinforcement ribs in a pattern around the periphery of the bulbous handle section 116 and along substantially the entire extent thereof in order to support or reinforce the plastic material thickness of the device 1 at this section 116 and to provide a better grip when in use. The bulbous handle section 116 or bubble handle advantageously allows for a turbulent flow inside the internal suction passage 110, decreasing the risk of bodily fluids clogging inside the suction passage.

The friction enhancing surface texture are provided as longitudinally extending grooves 120 along the periphery of the main part of the bulbous handle section 116. Thus, drips of bodily fluids collected during surgery upon this section can be removed and easily led along the ribs back down into the wound in order to further improve the grip upon the device 1. Alternatively, the friction enhancing surface texture may be provided in a transversal, helical, elongated, punctual, and/or harlequin like or crosswise pattern. The reinforcement ribs may also be provided by differences in the material thickness or by the application of a different type of plastic or other reinforcing material, e.g. having different hardness and/or tensile modulus than the base material. Preferably, the ribs are provided in the base material itself, which assists in reducing the total weight of the device 1. The bulbous handle section combined with the reinforcement ribs provide increased stability to the handle section and an ergonomically convenient firm grip during use in surgery and other bodily fluid creating operations.

Alternatively, the friction enhancing surface texture may be provided entirely as part of variations in the surface texture, or as tube material with varying thickness, or as different material than the base material. The number of pattern creating grooves or ribs may vary depending on type of application, dimension, i.e. diameter of device, and material being used; in fig. 1A the number shown is ten longitudinally extending grooves. Further the extent of the friction enhancing surface texture along the bulbous handle section 116 may also vary, depending upon the same parameters as just mentioned, but also upon required appearance and dimension, i.e. both diameter and length, of the bulbous handle section 116 itself.

The main part of the internal suction passage 110 of the bulbous handle section 116 is provided with a substantially circular cross-section to ease the removal of bodily fluids, but may alternatively be provided with other shapes, such as rectangular, semicircular, elliptical, or quadratic cross sections. The provision of longitudinally extending grooves 120 in the plastic material itself, i.e. the entire tube wall in the bulbous handle section 116 having a substantially uniform material thickness, may increase the turbulence chamber effect of the bulbous handle section and in particular provides the possibility of reducing the total weight of the device even further.

Preferably, the device 1 is made of a styrene butadiene copolymer or SBC, preferably Styrolux®, a registered trademark to BASF. Alternative clear styrene butadiene copolymers which may be used include Finaclear® 609 and K-resin® KR 01. Other clear types of plastic materials may alternatively be used such as cellulose proprionates, cellulose acetate butyrates, polypropylenes, polyethylenes, thermoplastic polyesters, polycarbonates, COG (cycloolefine), COC (cyclic olefin copolymers) such as TOPAS®, homopolymer/copolymers, PETG, nylons such as e.g. Grilamid® TR 90, polyamides such as e.g. Trogamid®CX 7323, polyurethanes such as Isoplast® 101 and combinations thereof. Clarity of the material used in the device is not substantial to the present invention, but merely advantageous to its function, and thus alternatively, other not as clear types of plastics materials are conceivable.

The selection of plastic material used during production depends upon different considerations, in this case in particular in order to provide a relatively high flexibility of at least the suction tip section 112 as well as a relatively stable bulbous handle section 116, in spite of the fact that the latter section may have a particularly thin material thickness due to the production method used and/or material used and/or surface texture used. If this friction enhancing surface texture was not provided, the material thickness in the bulbous handle section 116 would in many cases be too low to provide a stable handle, i.e. it would bend when being subjected to a relatively low force during flexing of the suction tip section 112.

Further, the material being selected for the device 1 has preferably a high transparency in order to ease inspection of the bodily fluids being sucked up by the surgical suction device during use. This property may be expressed in a high transmittance, which may be determined in a procedure, which will be described below. Further, it is advantageous to keep the device reflectance low, reducing glare from the device during use, which is reflected operation light in the device. This may be expressed by a low haze property, which also will be described below. Preferably, the device 1 is transparent and has a transmittance of in the order of 80 to 99 %, preferably from 85 to 98 %, and a low haze in the order of from 0 to 8%, preferably from 0 to 5% when performing the determination according to the standard of ASTM D 1003. According to the manufacturers data sheets Styrolux® has a transparency of 90% and a haze of 1,5% (at 2 mm), Finaclear® has a transparency of 90 to 93% and a haze of 3%, while a polypropylene such as the Exxon Escorene® PP 9074 (at 1 mm) has a haze of approx. 8%.

By making the device 1 of a styrene butadiene copolymer such as Styrolux®, it is possible to achieve a hardness of the plastic material in the order of from shore 60 to 80, preferably from shore 65 to 70 according to the standard ISO 868 shore D, which is presumed known to the skilled person, and further to achieve a tensile modulus of the plastic material is in the order of from 1300 to 1700 MPa, preferably from 1400 to 1500 MPa according to the standard ISO 527, which is also presumed known to the skilled person. As an example Styrolux® exhibit a tensile modulus of 1500 MPa and a shore D hardness of 68. Thus, a device 1 is provided, which exhibit excellent tensile modulus and hardness, which in combination results in at least a flexible suction tip section 112 and a stable and rigid handle section 116.

Due to the relatively low density and high hardness and tensile modulus of the styrene butadiene copolymer such as Styrolux® used in a surgical suction device 1 according to the invention, this results in a low total weight of such device 1. For example, a standard size device 1 in Styrolux® (length approx. 25 cm) is approx. four times lighter than such rigid device in PVC, i.e. 6 g and 25-30 g, respectively.

The styrene butadiene copolymers such as Styrolux® further exhibit excellent printability, allowing for printed messages or symbols into the device surface and/or sides.

Also, the use of a styrene butadiene copolymer reduces the dependence of PVC, which due to environmental considerations is being phased out, while still providing a relatively stable handle section for the device 1.

In fig.1B it is shown that the device 1 is a Yankauer provided with a bend 130 and a suction control port 140 in the form of a hole in the tube in the bulbous handle section 116 for when in use to be closed or opened controllably by a finger of the operator in order to control the suction force at the suction tip 112A. The bend 130 allows for increased control during surgery of the positioning of the suction tip 112A inside e.g. the wound and is shown in fig. 1A having a bend of around 45°, but may alternatively be at an angle of between 1° and 180°, depending on application and dimensions of the device 1.

Further, the device 1 is provided with four suction tip side holes 150, as is seen in fig. 1A to increase the suction efficiency of the device 100, and with a five fold ribbed male connector 160 at the connection section 116 to fix the device 100 firmly inside a flexible fluid removal tube (not shown) leading to a suction source (not shown). Obviously, any suitable number and position of the suction tip side holes may be provided, as well as any other suitable connector means.

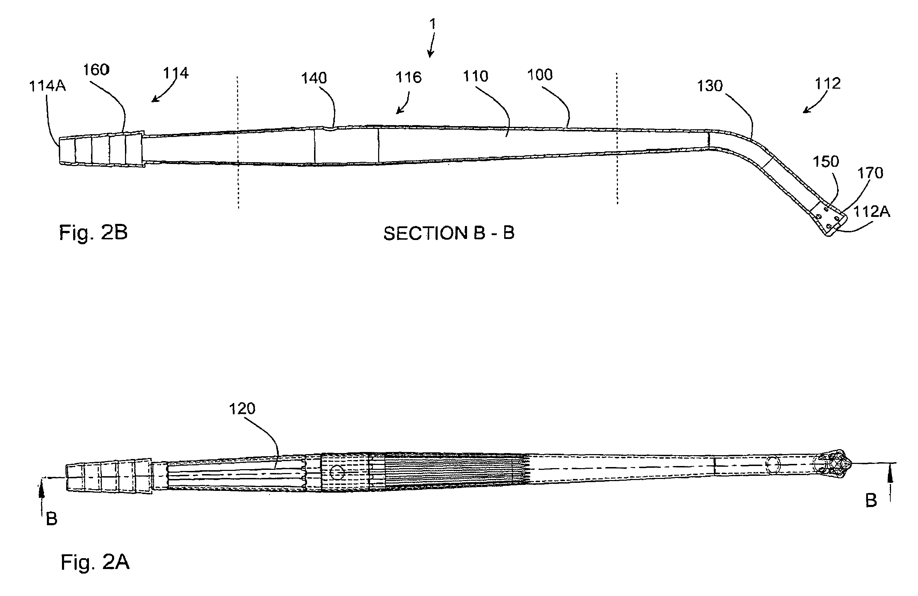

In fig. 2A is shown a second embodiment of a device according to the invention, which is shown in fig. 2B in a section along the line B-B in fig. 2A, where similar features are assigned like reference number as in fig. 1A and will not be described further. The device is in this case provided with a sump tip 170 at its distal end or suction tip section 112. This reduces the risk of tissue damage and increases the fluid flow volume and suction efficiency during use. Alternatively, the suction tip 112A may be rounded at its edge.

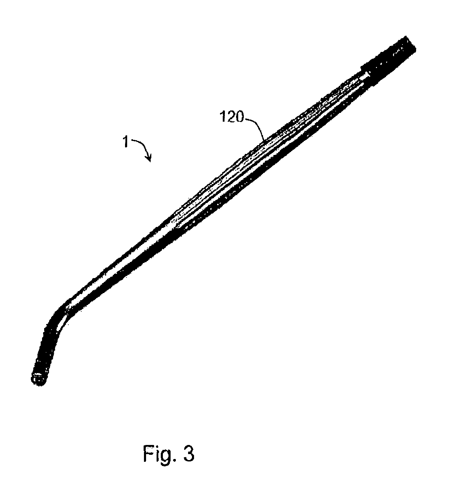

In fig. 3 is shown a perspective view of the first embodiment of the device 1 of fig. 1A and 1B. The longitudinally extending grooves 120 are clearly visible, being ten in total, providing an excellent grip when using gloves during surgery, and further providing a sufficient reinforcement when being made from a relatively soft plastic material such as a styrene butadiene copolymer such as Styrolux®.

A method for producing a surgical suction device may comprise the following steps: injection moulding a suction tube terminating at a suction tip section at the distal end thereof and a suction connection section at the proximal end thereof for connection to a suction source, and blow moulding a bulbous handle section while fixating at least the suction tip section and/or the suction connection section, in such a way that part of the suction tube is provided with a plurality of reinforcement ribs extending along at least a part of said bulbous handle section.

Injection blow moulding is a process, which first step enables to injection mould fine details in areas of the device, which areas are not subjected to blow moulding in the second step. The injection moulding may be substituted by extrusion or by other plastic forming techniques. After this moulding step, the device is provided with a blow moulded part having details that are only possible to form with injection moulding.

In the first step of the method an elongated test tube like formation is injection moulded with fine details such as e.g. bend 130, sump tip 170, connector part 160, even smaller holes like the suction tip side holes 150, and optionally signs (not shown), such as letters, numbers, production date, name of manufacturer, size, suction capacity, plastic material used, etc, in the suction connection section 114 and/or the suction tip section 112. Some or all of the details may optionally be provided after the injection moulding step, either in the following blow moulding step or by further processing. Moulding the device integrally significantly reduces the required production time.

In the second step of the method the elongated test tube like formation while still hot is blow moulded at least in that part of the formation, which is to form the bulbous handle section of the device, while at the same time the finely detailed areas are fixated during the duratiou of the blow moulding step. According to the invention, the surgical suction device is integrally blow moulded in such a way, that at least part of it is provided with a bulbous handle section 116 provided with a friction enhancing surface texture extending along at least a part thereof. This step may be performed by blow moulding inside a mold having form of the ribs and the bulbous handle section in reverse. The mold may be in cyclic steps close around successive elongated test tube like formations, and the blow pressure may be applied in a balloon like fashion to create the suction passages 110 therein.

After injection blow moulding the device, a suction port 140 can be formed by any given process, such as machining. Accordingly, a method is disclosed for producing a surgical suction device according to the invention, which is time reducing and produces a ready-to-use device by integrally moulding said device in an injection blow moulding step. Further, the method allows for a device with fine details having a low weight and a flexible suction tip section provided integrally in simple steps.

The flexibility of the suction tip section is a function of the length, diameter, and material thickness of the device and the hardness and tensile modulus of the plastic material used. The devices may be of different length and sizes depending on application, but in the case of the two preferred embodiments shown in fig 1A and 2A of standard size Yankauers (20-30 cm in length, 1-2 cm in diameter) made of Styrolux® the hardness and the tensile modulus is preferably between shore 60 and 80, and between 1300 and 1700 MPa, respectively.

This provides for a durable transparent, non-reflective Yankauer, which is provided with the advantages of the prior art flexible Yankauers having a flexible tip and of the prior art rigid Yankauers having a stable and rigid handle, but does not suffer the disadvantages of the same.

### Measurement of haze and transmittance

Haze is the scattering of light by a film that results in a cloudy appearance or poorer clarity of objects when viewed through the film. More technically, haze is the percentage of light transmitted through a film that is deflected by more than 2.5° (degrees) from the direction of the incoming beam. This property is used to describe transparent and translucent films, not opaque films, where a translucent material transmits light, but also diffuses it, so that objects cannot be seen clearly through it.

Film clarity is a highly desirable feature for the present application, because the type, amount and composition of the bodily fluids sucked up by a surgical suction device such as the device according to the present invention is important to estimate suction effectiveness and performance.

Haze is greatly influenced by materials selections and product design. Resin characteristics, such as crystallinity and molecular weight distribution, have a key impact. Copolymers are generally hazier than homopolymers. Additives and coatings usually contribute to increased haze. All other things being equal, thicker films will be hazier than thinner films. Additional variables, like process temperatures in the different stages of film-making, can further affect haze, so they are tightly controlled.

Haze is a property, which may be tested using a standard test procedure, such as the ASTM D 1003. Commercial hazemeters, such as the BKY-Gardner XL-211 Haze-gard and Haze-gard plus are typically used for this testing, which meters may also be used for transmittance testing. In the hazemeter, a unidirectional light beam is directed onto the film specimen, after which it enters into an integrating sphere, where a photo detector measures the total light transmitted by the film and the amount of transmitted light being scattered more than 2.5°. Haze is the percentage of total transmitted light that is scattered by more than 2.5°. A gate in the sphere controls the passage of light that deviates up to 2.5° from the incident beam.

Transmittance is the percentage of incident light that passes through a film.

By measuring in four positions, using the specimen and a reflectance standard, the ASTM D 1003 standard haze property and the transmittance are determined.

## Claims

1. A surgical suction device (1), comprising a suction tube (100) having a substantially longitudinally extending internal suction passage (110), terminating at a suction tip section (112) at the distal end thereof and a suction connection section (114) at the proximal end thereof for connection to a suction source;
**characterized in that**
the surgical suction device (1) is integrally blow moulded in a plastic material in such a way, that at least part of it is provided with a bulbous handle section (116) provided with a friction enhancing surface texture extending along at least a part thereof.

2. A surgical suction device according to claim 1 which is integrally injection blow moulded.

3. A surgical suction device according to claim 1 or 2, where the friction enhancing surface texture is provided as a plurality of reinforcement ribs in a pattern around the periphery of the bulbous handle section.

4. A surgical suction device according to any of the preceding claims, where the friction enhancing surface texture is longitudinally extending grooves (120) provided in the plastic material itself.

5. A surgical suction device according to any of the preceding claims, where the plastic material is a styrene butadiene copolymer, preferably Styrolux®.

6. A surgical suction device according to any of the preceding claims, where the hardness of the plastic material is in the order of from shore 60 to 80, preferably from shore 65 to 70 according to ISO 868 shore D.

7. A surgical suction device according to any of the preceding claims, where the tensile modulus of the plastic material is in the order of from 1300 to 1700 MPa, preferably from 1400 to 1500 MPa according to ISO 527.

8. A surgical suction device according to any of the preceding claims, where at least part of the internal suction passage (110) in the bulbous handle section (116) has a diameter, which is larger than that of the remaining part of the internal suction passage (110).

9. A surgical suction device according to any of the preceding claims, where the material thickness of the surgical suction device (1) is substantially uniform throughout the extent thereof, in particular in the bulbous handle section provided with the friction enhancing surface texture.

10. A surgical suction device according to any of the preceding claims, where the plastic material is transparent and has a transmittance of in the order of 80 to 99 %, preferably from 85 to 98 %, and a haze in the order of from 0 to 8%, preferably from 0 to 5% according to ASTM D 1003.

11. A surgical suction device according to any of the preceding claims, being a Yankauer provided with a bend (130) at the suction tip section (112) in the interval from 1 to 180°, and a suction control port (140) in the handle section (116).

12. A surgical suction device according to any of the preceding claims, further being provided with at least one suction tip side hole (150) in the suction tip section (112).

13. A surgical suction device according to any of the preceding claims, further being provided with an externally ribbed connector (160) in the suction connection section (114).

14. A surgical suction device according to any of the preceding claims, further being provided with a sump tip (170) at the suction tip section (112) provided with at least one suction tip side hole (150).
